# EUROPEAN PATENT SPECIFICATION

(11) **EP 4 074 249 B1**
(45) Date of publication and mention of the grant of the patent: **12.11.2025**
(21) Application number: 22168551.4
(22) Date of filing: 14.04.2022
(51) Int. Cl.: A61B 5/00, A61B 5/11, A61B 5/0205, A63B 24/00

(54) **METHOD AND DETECTING DEVICE FOR DETERMINING TIREDNESS OF USER**
VERFAHREN UND DETEKTIONSVORRICHTUNG ZUM BESTIMMEN DER MÜDIGKEIT EINES BENUTZERS
PROCÉDÉ ET DISPOSITIF DE DÉTECTION POUR DÉTERMINER LA FATIGUE DE L'UTILISATEUR

(30) Priority: 16.04.2021 TW 110113689
(43) Date of publication of application: 19.10.2022
(73) Proprietor: National Chung Hsing University, Taichung City 402 (TW)
(72) Inventor: LIN, Kuan-Jiuh, Taichung City 402 (TW); SU, Jun Wei, Chiayi City 600 (TW)
(74) Representative: Horak, Michael

(56) References cited:
- EP-B1- 3 001 864
- WO-A1-2015/140338
- JP-A- 2018 033 565
- US-A1- 2016 374 569

## Description

The disclosure relates to a method and a device for determining tiredness of a user according to human physiological information.

As smart wearable devices become more and more popular, smart watches and smart bracelets are now accessories that many people wear on a daily basis, and a lot of these people also use the physiological data recorded by the smart watches or bracelets as a reference for personal health management.

A conventional smart watch usually records heart rate as the basis for subsequent analysis of exercise time and calorie consumption. However, as the heart rate is being measured and recorded, the conventional smart watch is unable to provide feedback (e.g., excessive tiredness) in real time to the user, and thus the user cannot suitably adjust or stop the exercise in time. The user can only know his/her physical state when checking the conventional smart watch later.

A conventional assisting system for mobile vehicles, as disclosed in Taiwanese Utility Model Patent No. M585412, includes a driver condition detecting device and a warning device. The driver condition detecting device includes a heart rate detecting module, a storage module and a computing module. The heart rate detecting module is used for detecting heart rate of a driver or change in heart rate of the driver, and can be disposed on a wearable device to be worn by the driver.

Other prior art patent documents are also known, such as JP 2018-33565, which discloses an exercise support system, an exercise support method, and an exercise support device that allow a user to check a degree of tiredness.

Although the conventional assisting device can issue a notification when the heart rate of the user is outside of a predetermined range, it is prone to making inappropriate notifications when the user is exercising.

Therefore, an object of the disclosure is to provide a method for determining tiredness of a user that can alleviate at least one of the drawbacks of the prior art.

According to one embodiment of the disclosure, the method is implemented by a detecting device that stores a plurality of exercise modes respectively defined by a plurality of first ranges of heart-rate-related ratios, and each of the exercise modes has a variation threshold and a predetermined exercise time period. The method includes steps of:
A) obtaining, from a heart rate measuring unit, a heart rate of the user at successive time instances so as to result in a plurality of heart rate measurements; and
B) for each of the heart rate measurements, calculating a ratio of a resulting value of the heart rate measurement minus a resting heart rate of the user to a resulting value of a maximum heart rate of the user minus the resting heart rate to serve as a reference ratio;
C) selecting one of the exercise modes based on a last reference ratio;
D) obtaining, from a positioning unit, a plurality of speed measurements of the user that were successively measured in the predetermined exercise time period of the exercise mode selected in Step C;
E) calculating an average of the speed measurements to serve as an average speed;
F) dividing a number of those of the speed measurements that are greater or smaller than the average speed at least by a variation ratio by a total number of the speed measurements to obtain an abnormal speed ratio;
G) determining whether the average speed is greater than a predetermined speed and whether the abnormal speed ratio is smaller than a predetermined ratio;
H) when the determination on whether the average speed is greater than the predetermined speed and the determination on whether the abnormal speed ratio is smaller than the predetermined ratio are both affirmative, determining whether the last calculated reference ratio is greater than a previously calculated reference ratio by the variation threshold of the exercise mode selected in Step C, the previously calculated reference ratio being the reference ratio calculated at an earlier time before the time of calculating the last reference ratio and corresponding to the predetermined exercise time period of the exercise mode selected in Step C; and
I) outputting, to a display unit, a first notification indicating that the user is tired when the determination made in step H) is affirmative;
the method further comprising the following steps before Step C:
J) obtaining a plurality of acceleration measurements of the user in a predefined time period;
K) determining whether all the acceleration measurements in the predefined time period are smaller than a predetermined acceleration threshold;
L) when it is determined that all the acceleration measurements in the predefined time period are smaller than the predetermined acceleration threshold, determining tiredness of the user and outputting a second notification; and
M) when it is determined that not all the acceleration measurements in the predefined time period are smaller than the predetermined acceleration threshold, executing Steps C to I.

Another object of the disclosure is to provide a detecting device for monitoring heart rate of a user.

According to one embodiment of the disclosure, the monitoring device includes a heart rate measuring unit, a positioning unit, a display unit and a processing unit.

The heart rate measuring unit is configured to measure heart rate of the user at successive time instances so as to result in a plurality of heart rate measurements.

The positioning unit is configured to detect where the user is located at successive time instances so as to obtain a plurality of positions, and is further configured to calculate a plurality of speed measurements of the user based on the positions.

The processing unit is electrically connected to the heart rate measuring unit, the positioning unit and the display unit, and stores a plurality of exercise modes respectively defined by a plurality of ranges of heart-rate-related ratios. Each of the exercise modes has a variation threshold and a predetermined exercise time period. The processing unit is configured to implement a tiredness-determining procedure including steps of:
a) obtaining the heart rate measurements of the user from said heart rate measuring unit,
b) for each of the heart rate measurements, calculating a ratio of a resulting value of the heart rate measurement minus a resting heart rate of the user to a resulting value of a maximum heart rate of the user minus the resting heart rate to serve as a reference ratio,
c) selecting one of the exercise modes based on a last reference ratio,
d) obtaining, from said positioning unit (3), a plurality of speed measurements that were successively measured by said positioning unit (3) in the predetermined exercise time period of the exercise mode selected in step c,
e) calculating an average of the speed measurements to serve as an average speed,
f) dividing a number of those of the speed measurements that are greater or smaller than the average speed at least by a variation ratio by a total number of the speed measurements to obtain an abnormal speed ratio,
g) determining whether the average speed is greater than a predetermined speed and whether the abnormal speed ratio is smaller than a predetermined ratio,
h) when the determination on whether the average speed is greater than the predetermined speed and the determination on whether the abnormal speed ratio is smaller than the predetermined ratio are both affirmative, determining whether the last calculated reference ratio is greater than a previously calculated reference ratio by the variation threshold of the exercise mode selected in step c, the previously calculated reference ratio being the reference ratio calculated at an earlier time before the time of calculating the last reference ratio and corresponding to the predetermined exercise time period of the exercise mode selected in Step c, and
i) controlling said display unit to output a first notification indicating that the user is tired when the determination made in step h) is affirmative.

An acceleration measuring unit is electrically connected to said processing unit and configured to successively measure acceleration measurements of the user, and the tiredness-determining procedure implemented by said processing unit further includes steps, before step c, of:
obtaining, from said acceleration measuring unit, a plurality of acceleration measurements of the user measured in a predefined time period;
determining whether all the acceleration measurements in the predefined time period are smaller than a predetermined acceleration threshold;
when it is determined that all the acceleration measurements in the predefined time period are smaller than the predetermined acceleration threshold, determining tiredness of the user and controlling said display unit to output a second notification; and
when it is determined that not all the acceleration measurements in the predefined time period are smaller than the predetermined acceleration threshold, executing steps c to i of the tiredness-determining procedure.

The invention is defined by the appended claims 1-14.

Other features and advantages of the disclosure will become apparent in the following detailed description of the embodiments with reference to the accompanying drawings, of which:
Figure 1 is a flow chart illustrating steps of a method for determining tiredness of a user according to an embodiment of the disclosure;
Figure 2 is a block diagram of a detecting device for determining tiredness of the user according to an embodiment of the disclosure;
Figure 3 is a chart of heart rate measurements, for illustrating an example of determining exercise modes according to an embodiment of the disclosure; and
Figure 4 is a chart of heart rate measurements, for illustrating an example of determining tiredness levels when the user is resting according to an embodiment of the disclosure.

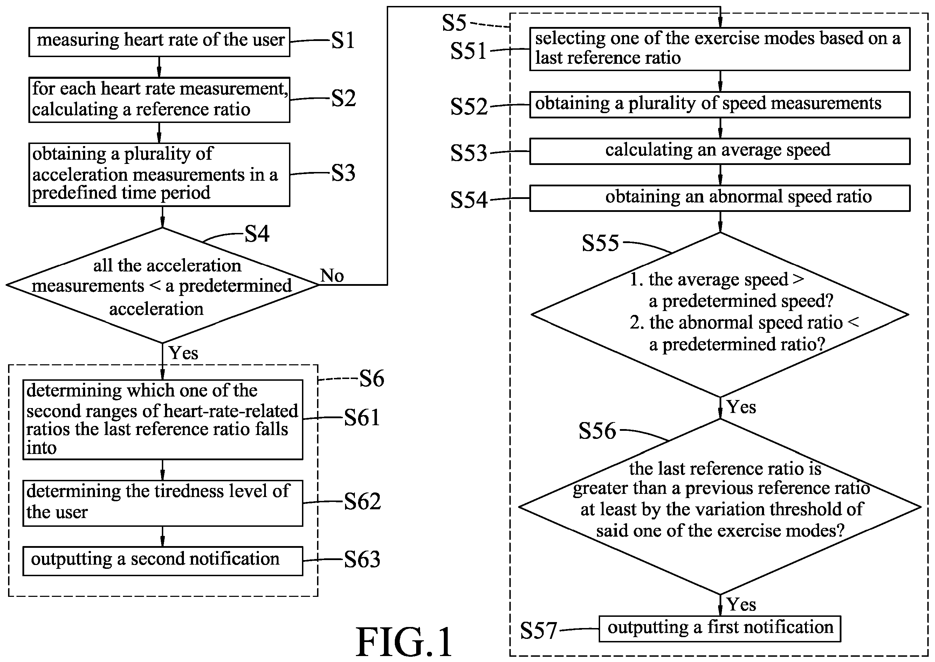

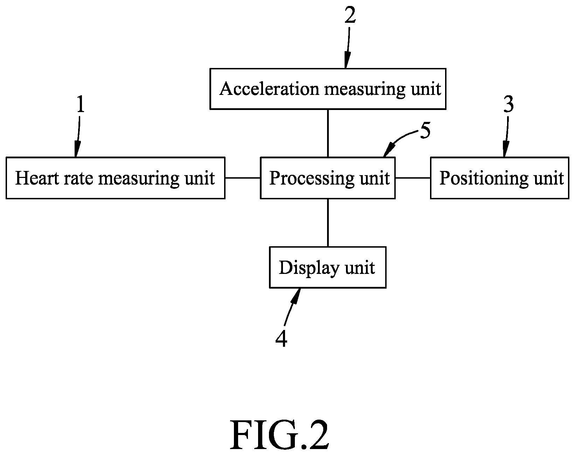

Figure 1 is a flow chart illustrating steps of a method for determining tiredness of a user according to one embodiment of the disclosure. The method is implemented by a detecting device shown in Figure 2.

Referring to Figure 2, the detecting device includes a heart rate measuring unit 1, an acceleration measuring unit 2, a positioning unit 3, a display unit 4 and a processing unit 5. The detecting device may be, but not limited to, a wearable device worn by the user.

The heart rate measuring unit 1 is configured to measure heart rate of the user at successive time instances so as to result in a plurality of heart rate measurements. In this embodiment, the heart rate measuring unit 1 is an optical heart rate sensor.

The acceleration measuring unit 2 is configured to successively measure a plurality of acceleration measurements of the user. The acceleration measuring unit 2 may be, but not limited to, an accelerometer.

The positioning unit 3 is configured to detect where the user is located at successive time instances so as to obtain a plurality of positions, and is further configured to calculate a plurality of speed measurements of the user based on the positions. For example, the positioning unit 3 includes a global positioning system (GPS) sensor for obtaining the position of the user, and a computing device (e.g., a processor, a mobile processor, a microprocessor, a microcontroller, etc.) for calculating the speed measurements.

The display unit 4 is configured to output a notification. In this embodiment, the display unit 4 is, but not limited to, a display screen.

The processing unit 5 is electrically connected to the heart rate measuring unit 1, the acceleration measuring unit 2, the positioning unit 3 and the display unit 4. The processing unit 5 stores a plurality of exercise modes and a plurality of tiredness levels. The exercise modes are respectively defined by a plurality of first ranges of heart-rate-related ratios. The tiredness levels are respectively defined by a plurality of second ranges of heart-rate-related ratios. Each of the exercise modes has a variation threshold and a predetermined exercise time period.

In this embodiment, the processing unit 5 stores five exercise modes, and details thereof are shown in Table 1 below.

**Table 1**

| Exercise Mode | Definition | Predetermined Exercise Time Period | variation Threshold |
|---|---|---|---|
| Exercise Mode 1 | reference ratio ≤ 30% | 120 minutes | 10% |
| Exercise Mode 2 | 30% < reference ratio ≤ 50% | 90 minutes | 10% |
| Exercise Mode 3 | 50% < reference ratio ≤ 70% | 30 minutes | 10% |
| Exercise Mode 4 | 70% < reference ratio ≤ 90% | 10 minutes | 10% |
| Exercise Mode 5 | 90% < reference ratio | 5 minutes | 10% |

In this embodiment, the processing unit 5 stores five tiredness levels, and details thereof are shown in Table 2 below.

**Table 2**

| Tiredness Level | Definition |
|---|---|
| not tired | reference ratio ≤ 2% |
| slightly tired | 2% < reference ratio ≤ 5% |
| tired | 5% < reference ratio ≤ 8% |
| very tired | 8% < reference ratio ≤ 11% |
| extremely tired | 11% < reference ratio |

The processing unit 5 is configured to implement a tiredness-determining procedure for determining tiredness of the user based on heart rate measurements, the acceleration measurements and the speed measurements of the user.

The processing unit 5 may be embodied using a central processing unit (CPU), a microprocessor, a microcontroller, a single core processor, a multi-core processor, a dual-core mobile processor, a microprocessor, a microcontroller, a digital signal processor (DSP), a field-programmable gate array (FPGA), an application specific integrated circuit (ASIC), a radio-frequency integrated circuit (RFIC), a system on chip (SoC), etc.

The method for determining tiredness of the user (i.e., the tiredness-determining procedure implemented by the processing unit 5) according to one embodiment includes the following steps.

In step S1, the processing unit 5 obtains a plurality of heart rate measurements of the user that were successively measured by the heart rate measuring unit 1.

In step S2, for each of the heart rate measurements, the processing unit 5 calculates a ratio of a resulting value of the heart rate measurement minus a resting heart rate of the user to a resulting value of a maximum heart rate of the user minus the resting heart rate to serve as a reference ratio.

The maximum heart rate is an upper limit of what a person's cardiovascular system can typically handle during exercise (i.e., the fastest rate that the heart is capable of beating), and generally decreases with age. The maximum heart rate may be estimated by subtracting age from 220. The resting heart rate is a normal heart rate when a person is at rest. The resting heart rates of children over 10 years old and adults including seniors range from 60 to 100 beats per minute. The resting heart rates of well-trained adult athletes range from 40 to 60 beats per minute. In this embodiment, the maximum heart rate and the resting heart rate may be input by the user. In some embodiments, the processing unit 5 may pre-store a plurality of reference maximum heart rates and a plurality of reference resting heart rates that correspond respectively to the reference maximum heart rates, and each pair of one of the reference maximum heart rates and the corresponding one of the reference resting heart rates corresponds respectively to different ages; the processing unit 5 selects one of the reference maximum heart rates and the corresponding one of the reference resting heart rates according to an age inputted by the user and uses the reference maximum heart rate and reference resting heart rate thus selected as the maximum heart rate and the resting heart rate of the user. In some embodiments, the resting heart rate may be obtained by taking an average of heart rate measurements measured by the heart rate measuring unit 1 when the acceleration of the user continues to be smaller than a predetermined value (e.g., 20 cm/s²) for a predetermined time period (e.g., 20 minutes).

In step S3, the processing unit 5 obtains, from the acceleration measuring unit 2, a plurality of acceleration measurements of the user measured in a predefined time period. In this embodiment, the predefined time period is 20 minutes.

In step S4, the processing unit 5 determines whether all the acceleration measurements in the predefined time period are smaller than a predetermined acceleration threshold. When it is determined that all the acceleration measurements are smaller than the predetermined acceleration threshold, the flow goes to step S6; otherwise, the flow goes to step S5. In this embodiment, the predetermined acceleration threshold is 20 cm/s².

It should be noted that step S4 is used to determine whether the user is exercising or resting. The user is determined to be resting when it is determined that all the acceleration measurements are smaller than the predetermined acceleration threshold.

When it is determined that not all the acceleration measurements in the predefined time period are smaller than the predetermined acceleration threshold, in step S5, the processing unit 5 determines tiredness of the user during exercise. Specifically, step S5 includes sub-steps S51 to S57.

In sub-step S51, the processing unit 5 selects one of the exercise modes based on the reference ratio that was calculated in step S2 based on a last one of the heart rate measurements (hereinafter referred to as "the last reference ratio"). Specifically, the processing unit 5 first determines which one of the first ranges of heart-rate-related ratios the last reference ratio falls into, and then selects one of the exercise modes that corresponds to one of the first ranges of heart-rate-related ratios, into which the last reference ratio falls.

In sub-step S52, the processing unit 5 obtains a plurality of speed measurements of the user that were successively measured by the positioning unit 3 in a past time period that has a length equal to the predetermined exercise time period of said one of the exercise modes.

In sub-step S53, the processing unit 5 calculates an average of the speed measurements obtained in sub-step S52 to serve as an average speed.

In sub-step S54, the processing unit 5 divides a number of those of the speed measurements that are greater or smaller than the average speed at least by a variation ratio by a total number of the speed measurements, so as to obtain an abnormal speed ratio. In this embodiment, the variation ratio is, but not limited to, 20%.

In sub-step S55, the processing unit 5 determines whether the average speed is greater than a predetermined speed and whether the abnormal speed ratio is smaller than a predetermined ratio. When the determination on whether the average speed is greater than the predetermined speed and the determination on whether the abnormal speed ratio is smaller than the predetermined ratio are both affirmative, the flow goes to sub-step S56; otherwise, the method is terminated. In this embodiment, the predetermined speed is 10 km/h, and the predetermined ratio is 1/6, but not limited thereto.

It should be noted that, in sub-step S55, determining whether the average speed is greater than the predetermined speed is to determine whether the user is moving places **(e.g.,** doing an exercise such as jogging or cycling), and determining whether the abnormal speed ratio is smaller than the predetermined ratio is to avoid determining tiredness of the user when the speed measurements reveal an excessive change in speed. That is to say, sub-step S55 can avoid determining tiredness of the user when the user is in the midst of speeding up or slowing down, so as to reduce probability of making an incorrect determination on tiredness of the user.

When the average speed is greater than the predetermined speed and the abnormal speed ratio is smaller than the predetermined ratio, in sub-step S56, the processing unit 5 determines whether the last reference ratio is greater than a previous one of the reference ratios (hereinafter referred to as "previous reference ratio") at least by the variation threshold of said one of the exercise modes, wherein the previous reference ratio is calculated based on one of the heart rate measurements that was measured earlier than said last one of the heart rate measurements by the predetermined exercise time period of said one of the exercise modes. When it is determined that the last reference ratio is greater than the previous reference ratio at least by the variation threshold of said one of the exercise modes, the flow goes to sub-step S57; otherwise, the method is terminated.

In sub-step S57, the processing unit 5 controls the display unit 4 to output a first notification indicating that the user is tired.

When it is determined in step S4 that all the acceleration measurements are smaller than the predetermined acceleration threshold, in step S6, the processing unit 5 determines tiredness of the user when resting. Specifically, step S6 includes sub-steps of S61 to S63.

In sub-step S61, the processing unit 5 determines which one of the second ranges of heart-rate-related ratios the last reference ratio falls into.

In sub-step S62, the processing unit 5 determines that the user is at one of the tiredness levels that corresponds to one of the second ranges of heart-rate-related ratios, into which the last reference ratio falls.

In sub-step S63, the processing unit 5 controls the display unit 4 to output a second notification that corresponds to said one of the tiredness levels.

The method of the disclosure may be repeated to continuously determine tiredness of the user. It should be noted that steps/sub-steps of the method are not necessarily implemented in the order given above, and some of the steps/sub-steps may be implemented simultaneously.

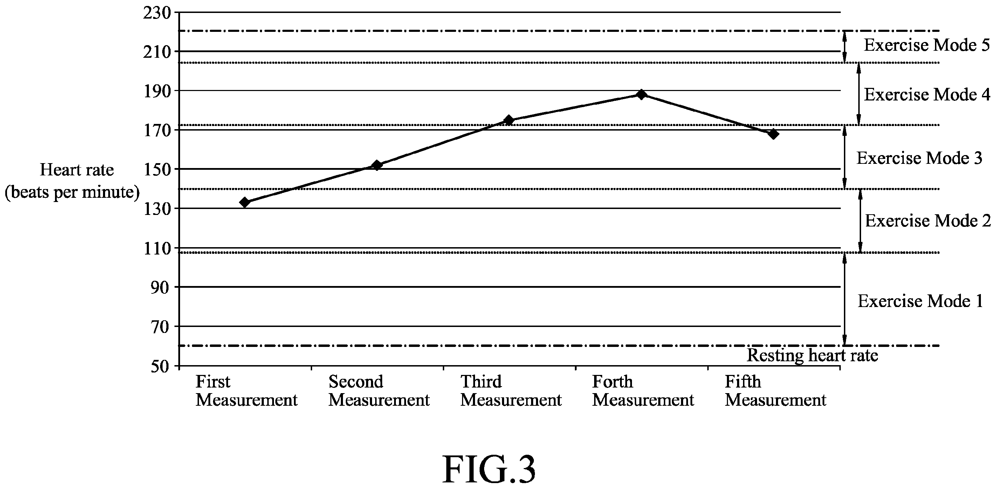

Referring to Figures 1 and 3 and Table 3 below, an exemplary flow for determining one of the exercise modes is described in the following. When it is determined in step S4 that at least one of the acceleration measurements of the user in the predefined time period (20 minutes) is greater than the predetermined acceleration threshold (20 cm/s²), and it is determined in sub-step S55 that the average speed is greater than the predetermined speed (10 km/h) and the abnormal speed ratio is smaller than the predetermined ratio (1/6), the flow goes to sub-step S56 to determine whether the last reference ratio is greater than the previous reference ratio . For example, five heart rate measurements of 133, 152, 175, 188 and 168 are obtained in step S1 in sequence, and the maximum heart rate is 220 and the resting heart rate is 60. Accordingly, the reference ratios for the five heart rate measurements are 46%, 58%, 72%, 80% and 68% (step S2), respectively, and the exercise modes corresponding to the five heart rate measurements are Exercise Mode 2, Exercise Mode 3, Exercise Mode 4, Exercise Mode 4 and Exercise Mode 5 (see Table 1), respectively. It should be noted that there may be one or more heart rate measurements that were measured between any two of the first to fifth heart rate measurements and that are not shown in this example.

**Table 3**

| | First | Second | Third | Forth | Fifth |
|---|---|---|---|---|---|
| Heart Rate | 133 | 152 | 175 | 188 | 168 |
| Ref . Ratio | 46% | 58% | 72% | 80% | 68% |
| Exercise Mode | Mode 2 | Mode 3 | Mode 4 | Mode 4 | Mode 3 |

Taking the second heart rate measurement as an example, the exercise mode that corresponds to the second heart rate measurement is Exercise Mode 3 (with reference to Table 1), so sub-step S56 is to determine whether the reference ratio of the second heart rate measurement (58%) is greater than the previous reference ratio, which corresponds to a heart rate measurement that was measured 30 minutes (i.e., the predetermined exercise time period of Exercise Mode 3) earlier, at least by 10%. It is assumed that the first heart rate measurement of 133 was measured earlier than the second heart rate measurement of 152 by the predetermined exercise time period (i.e., 30 minutes), so the previous reference ratio is 46%. Compared with the previous reference ratio (46%), the reference ratio of the second heart rate measurement (58%) has an increase of 12%, which is more than 10% (the variation threshold of Exercise mode 3) . Accordingly, the display unit 4 of the detecting device outputs the first notification indicating that the user is tired (sub-step S57).

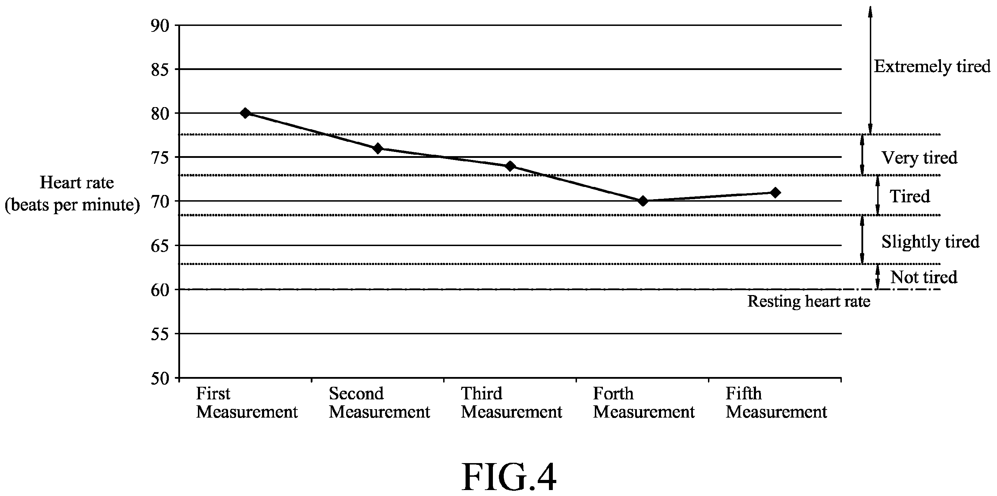

Referring to Figures 1 and 4 and Table 4, an exemplary flow for determining one of the tiredness levels of the user is described in the following. When all the acceleration measurements of the user in the predefined time period (20 minutes) are smaller than the predetermined acceleration threshold (20 cm/s²), the flow goes to step S6 to determine tiredness of the user when resting. For example, five heart rate measurements of 80, 76, 74, 70 and 71 are obtained in step S1 in sequence, and the maximum heart rate is 220 and the resting heart rate is 60. Accordingly, the reference ratios for the five heart rate measurements are 13%, 10%, 9%, 6%, and 7%, respectively (step S2), and the tiredness levels corresponding to the five heart rate measurements are "extremely tired", "very tired", "very tired", "tired" and "tired", respectively. Specifically, for the second heart rate measurement of 76, the reference ratio of 10% falls into the second range of heart-rate-related ratio "8% < reference ratio ≤ 11%" (see Table 2), and thus the tiredness level of the second heart rate measurement is determined as "very tired". It should be noted that there may be one or more heart rate measurements that were measured between any two of the first to fifth heart rate measurements and that are not shown in this example.

**Table 4**

| | First | Second | Third | Forth | Fifth |
|---|---|---|---|---|---|
| Heart Rate | 80 | 76 | 74 | 70 | 71 |
| Reference Ratio | 13% | 10% | 9% | 6% | 7% |
| Tiredness Level | extremely tired | very tired | very tired | tired | tired |

It should be noted that, unless otherwise specified, the use of the ordinal adjectives "first," "second," and "third," etc., to describe a common object, merely indicates that different instances of like objects are being referred to and does not intend to imply that the objects so described must be in a given sequence, either temporally, spatially, in ranking or in any other manner.

According to the above description, the embodiment of the disclosure provides a method and a detecting device for determining tiredness of a user. The advantages of the method and the detecting device are as follows. An exercise mode is first selected based on a reference ratio of a heart rate measurement, and thresholds for the subsequent determinations are dynamically determined according to the exercise mode, so that the likelihood of making incorrect determinations is reduced as compared to the case if uniform thresholds were used. Further, tiredness determination is made only under the condition that an average speed is greater than a predetermined speed and an abnormal speed ratio is smaller than a predetermined ratio, so as to avoid incorrectly determining tiredness of the user when the user is speeding up or slowing down. Moreover, the method first determines whether the user is exercising or resting based on acceleration measurements, and then adopts difference logical flows in determining whether the user is tired respectively during exercise and when resting, such that tiredness of the user thus determined is more in line with actual situation.

## Claims

1. A processor-implemented method of determining tiredness of a user, to be implemented by a detecting device that stores a plurality of exercise modes and a plurality of first ranges of heart-rate-related ratios, each of the exercise modes being defined by a first range of heart-rate-related ratios, and having a variation threshold and a predetermined exercise time period, the method comprising steps of:
A) -obtaining, from a heart rate measuring unit (1), a heart rate of the user at successive time instances so as to result in a plurality of heart rate measurements (S1); and
B) for each of the heart rate measurements, calculating a ratio of a resulting value of the heart rate measurement minus a resting heart rate of the user to a resulting value of a maximum heart rate of the user minus the resting heart rate to serve as a reference ratio (S2);
**characterized in that** the method further comprises the following steps after step B:
C) selecting one of the exercise modes based on the last calculated reference ratio (S51);
D) obtaining, from a positioning unit (3), a plurality of speed measurements of the user that were successively measured over a time period corresponding to the predetermined exercise time period of the exercise mode selected in Step C (S52);
E) calculating an average of the speed measurements to serve as an average speed (S53);
F) dividing a number of those of the speed measurements that are greater or smaller than the average speed at least by a variation ratio by a total number of the speed measurements to obtain an abnormal speed ratio (S54);
G) determining whether the average speed is greater than a predetermined speed and whether the abnormal speed ratio is smaller than a predetermined ratio (S55);
H) when the determination on whether the average speed is greater than the predetermined speed and the determination on whether the abnormal speed ratio is smaller than the predetermined ratio are both affirmative, determining whether the last calculated reference ratio is greater than a previously calculated reference ratio by the variation threshold of the exercise mode selected in Step C, the previously calculated reference ratio being the reference ratio calculated at an earlier time being before the time of calculating the last reference ratio and corresponding to the predetermined exercise time period of the exercise mode selected in Step C (S56); and
I) outputting, to a display unit (4), a first notification indicating that the user is tired during exercising when the determination made in step H) is affirmative (S57);
the method further comprising the following steps before Step C:
J) obtaining, from an acceleration measuring unit (2), a plurality of acceleration measurements of the user in a predefined time period (S3);
K) determining whether all the acceleration measurements in the predefined time period are smaller than a predetermined acceleration threshold (S4);
L) when it is determined that all the acceleration measurements in the predefined time period are smaller than the predetermined acceleration threshold, determining tiredness of the user during resting and outputting a corresponding second notification (S6); and
M) when it is determined that not all the acceleration measurements in the predefined time period are smaller than the predetermined acceleration threshold, executing Steps C to I.

2. The method of Claim 1, the detecting device further storing a plurality of tiredness levels and a plurality of second ranges of heart-rate-related ratios, each tiredness level being defined by a second range of heart-rate-related ratios, the method **characterized in that** step L) includes:
determining which one of the second ranges of heart-rate-related ratios the last calculated reference ratic falls into (S61); and
determining that the user is at one of the tiredness levels that corresponds to one of the second ranges of heart-rate-related ratios, into which the last calculated reference ratio falls (S62).

3. The method of any one of Claim 1 to 2, **characterized in that** the predefined time period is 20 minutes.

4. The method of any one of Claim 1 to 3, **characterized in that** the predetermined acceleration threshold is 20 cm/s².

5. The method of any one of Claim 1 to 4, **characterized in that** the variation ratio is 20%.

6. The method of any one of Claim 1 to 5, **characterized in that** the predetermined speed is 10 km/h, and the predetermined ratio is 1/6.

7. The method of any one of Claim 1 to 6, **characterized in that** the variation threshold of each of the exercise modes is 10%.

8. A detecting device for determining tiredness of a user, comprising:
a heart rate measuring unit (1) configured to measure heart rate of the user at successive time instances so as to result in a plurality of heart rate measurements;
a positioning unit (3) configured to detect where the user is located at successive time instances so as to obtain a plurality of positions, and further configured to calculate a plurality of speed measurements of the user based on the positions;
a display unit (4); and
a processing unit (5) electrically connected to said heart rate measuring unit (1), said positioning unit (3), and said display unit (4), and storing a plurality of exercise modes and a plurality of first ranges of heart-rate-related ratios, each of the exercise modes being defined by a first range of heart-rate-related ratios, and a variation threshold and a predetermined exercise time period, said processing unit (5) being configured to implement a tiredness-determining procedure that includes steps of
a) obtaining the heart rate measurements of the user from said heart rate measuring unit (1), and
b) for each of the heart rate measurements, calculating a ratio of a resulting value of the heart rate measurement minus a resting heart rate of the user to a resulting value of a maximum heart rate of the user minus the resting heart rate to serve as a reference ratio,
**characterized in that** the tiredness-determining procedure implemented by said processing unit (5) further comprises the following steps after step b:
c) selecting one of the exercise modes based on the last calculated reference ratio,
d) obtaining, from said positioning unit (3), a plurality of speed measurements that were successively measured by said positioning unit (3) over a time period corresponding to the predetermined exercise time period of the exercise mode selected in step c,
e) calculating an average of the speed measurements to serve as an average speed,
f) dividing a number of those of the speed measurements that are greater or smaller than the average speed at least by a variation ratio by a total number of the speed measurements to obtain an abnormal speed ratio,
g) determining whether the average speed is greater than a predetermined speed and whether the abnormal speed ratio is smaller than a predetermined ratio,
h) when the determination on whether the average speed is greater than the predetermined speed and the determination on whether the abnormal speed ratio is smaller than the predetermined ratio are both affirmative, determining whether the last calculated reference ratio is greater than a previously calculated reference ratio by the variation threshold of the exercise mode selected in step c, the previously calculated reference ratio being the reference ratio calculated at an earlier time being before the time of calculating the last reference ratio and corresponding to the predetermined exercise time period of the exercise mode selected in Step c, and
i) controlling said display unit (4) to output a first notification indicating that the user is tired during exercising when the determination made in step h) is affirmative; and
further **characterized by** an acceleration measuring unit (2) electrically connected to said processing unit (5) and configured to successively measure acceleration measurements of the user, the tiredness-determining procedure implemented by said processing unit (5) further includes steps, before step c, of:
obtaining, from said acceleration measuring unit (2), a plurality of acceleration measurements of the user measured in a predefined time period;
determining whether all the acceleration measurements in the predefined time period are smaller than a predetermined acceleration threshold;
when it is determined that all the acceleration measurements in the predefined time period are smaller than the predetermined acceleration threshold, determining tiredness of the user during resting and controlling said display unit (4) to output a corresponding second notification; and
when it is determined that not all the acceleration measurements in the predefined time period are smaller than the predetermined acceleration threshold, executing steps c to i of the tiredness-determining procedure.

9. The detecting device of Claim 8, **characterized in that** said processing unit (5) further stores a plurality of tiredness levels and a plurality of second ranges of heart-rate-related ratios, each tiredness level being defined by a second range of heart-rate-related ratios, and is configured to determine tiredness of the user by:
determining which one of the second ranges of heart-rate-related ratios the last calculated reference ratic falls into; and
determining that the user is at one of the tiredness levels that corresponds to one of the second ranges of heart-rate-related ratios, into which the last calculated reference ratio falls, and outputting the second notification that corresponds to said one of the tiredness levels.

10. The detecting device of any one of Claim 8 to 9, **characterized in that** the predefined time period is 20 minutes.

11. The detecting device of any one of Claim 8 to 10, **characterized in that** the predetermined acceleration threshold is 20 cm/s².

12. The detecting device of any one of Claim 8 to 11, **characterized in that** the variation ratio is 20%.

13. The detecting device of any one of Claim 8 to 12, **characterized in that** the predetermined speed is 10 km/h, and the predetermined ratio is 1/6.

14. The detecting device of any one of Claim 8 to 13, **characterized in that** the variation threshold of each of the exercise modes is 10%.

## Patentansprüche

1. Prozessorimplementiertes Verfahren zum Bestimmen der Müdigkeit eines Benutzers, das durch eine Detektionsvorrichtung implementiert wird, die eine Vielzahl von Trainingsmodi und eine Vielzahl von ersten Bereichen von Herzfrequenz-bezogenen Verhältnissen speichert, wobei jeder der Trainingsmodi durch einen ersten Bereich von Herzfrequenz-bezogenen Verhältnissen definiert ist und eine Variationsschwelle und einen vorbestimmten Trainingszeitraum aufweist, wobei das Verfahren die folgenden Schritte umfasst:
A) Erhalten einer Herzfrequenz des Benutzers zu aufeinanderfolgenden Zeitpunkten von einer Herzfrequenzmesseinheit (1), um eine Vielzahl von Herzfrequenzmessungen (S1) zu erzielen; und
B) Berechnen eines Verhältnisses eines resultierenden Werts der Herzfrequenzmessung abzüglich einer Ruheherzfrequenz des Benutzers zu einem resultierenden Wert einer maximalen Herzfrequenz des Benutzers abzüglich der Ruheherzfrequenz für jede der Herzfrequenzmessungen, um als Referenzverhältnis (S2) zu dienen;
**gekennzeichnet dadurch, dass** das Verfahren nach Schritt B ferner die folgenden Schritte umfasst:
C) Auswählen eines der Trainingsmodi basierend auf dem zuletzt berechneten Referenzverhältnis (S51);
D) Erhalten einer Vielzahl von Geschwindigkeitsmessungen des Benutzers, die nacheinander über einen Zeitraum gemessen wurden, der dem vorbestimmten Trainingszeitraum des in Schritt C (S52) ausgewählten Trainingsmodus entspricht, von einer Positionierungseinheit (3);
E) Berechnen eines Durchschnittswerts der Geschwindigkeitsmessungen, um als Durchschnittsgeschwindigkeit (S53) zu dienen;
F) Dividieren einer Anzahl derjenigen der Geschwindigkeitsmessungen, die um mindestens ein Variationsverhältnis größer oder kleiner als die Durchschnittsgeschwindigkeit sind, durch eine Gesamtzahl der Geschwindigkeitsmessungen, um ein abnormales Geschwindigkeitsverhältnis (S54) zu erhalten;
G) Bestimmen, ob die Durchschnittsgeschwindigkeit größer als eine vorbestimmte Geschwindigkeit ist und ob das abnormale Geschwindigkeitsverhältnis kleiner als ein vorbestimmtes Verhältnis (S55) ist;
H) wenn die Bestimmung, ob die Durchschnittsgeschwindigkeit größer als die vorbestimmte Geschwindigkeit ist, und die Bestimmung, ob das abnormale Geschwindigkeitsverhältnis kleiner als das vorbestimmte Verhältnis ist, beide positiv sind, Bestimmen, ob das zuletzt berechnete Referenzverhältnis um die Variationsschwelle des in Schritt C ausgewählten Trainingsmodus größer als ein zuvor berechnetes Referenzverhältnis ist, wobei das zuvor berechnete Referenzverhältnis das Referenzverhältnis ist, das zu einem früheren Zeitpunkt vor dem Zeitpunkt der Berechnung des letzten Referenzverhältnisses berechnet wurde und dem vorbestimmten Trainingszeitraum des in Schritt C (S56) ausgewählten Trainingsmodus entspricht; und
I) Ausgeben einer ersten Benachrichtigung, die anzeigt, dass der Benutzer während des Trainings müde ist, an eine Display-Einheit (4), wenn die in Schritt H) gemachte Bestimmung positiv ist (S57);
wobei das Verfahren vor Schritt C ferner die folgenden Schritte umfasst:
J) Erhalten einer Vielzahl von Beschleunigungsmessungen des Benutzers in einem vordefinierten Zeitraum (S3) von einer Beschleunigungsmesseinheit (2);
K) Bestimmen, ob alle Beschleunigungsmessungen in dem vordefinierten Zeitraum kleiner als eine vorbestimmte Beschleunigungsschwelle (S4) sind;
L) wenn bestimmt wird, dass alle Beschleunigungsmessungen in dem vordefinierten Zeitraum kleiner als die vorbestimmte Beschleunigungsschwelle sind, Bestimmen der Müdigkeit des Benutzers während der Ruhephase und Ausgeben einer entsprechenden zweiten Benachrichtigung (S6); und
M) wenn bestimmt wird, dass nicht alle Beschleunigungsmessungen in dem vordefinierten Zeitraum kleiner als die vorbestimmte Beschleunigungsschwelle sind, Ausführen der Schritte C bis I.

2. Verfahren nach Anspruch 1, wobei die Detektionsvorrichtung ferner eine Vielzahl von Müdigkeitsgraden und eine Vielzahl von zweiten Bereichen von Herzfrequenz-bezogenen Verhältnissen speichert, wobei jeder Müdigkeitsgrad durch einen zweiten Bereich von Herzfrequenz-bezogenen Verhältnissen definiert ist,
wobei das Verfahren **dadurch gekennzeichnet ist, dass** Schritt L) Folgendes enthält:
Bestimmen, in welchen der zweiten Bereiche von Herzfrequenz-bezogenen Verhältnissen das zuletzt berechnete Referenzverhältnis fällt (S61); und
Bestimmen, dass sich der Benutzer in einem der Müdigkeitsgrade befindet, der einem der zweiten Bereiche von Herzfrequenz-bezogenen Verhältnissen entspricht, in den das zuletzt berechnete Referenzverhältnis fällt (S62).

3. Verfahren nach einem der Ansprüche 1 bis 2, **dadurch gekennzeichnet, dass** der vordefinierte Zeitraum 20 Minuten beträgt.

4. Verfahren nach einem der Ansprüche 1 bis 3, **dadurch gekennzeichnet, dass** die vorbestimmte Beschleunigungsschwelle 20 cm/s² beträgt.

5. Verfahren nach einem der Ansprüche 1 bis 4, **dadurch gekennzeichnet, dass** das Variationsverhältnis 20 % beträgt.

6. Verfahren nach einem der Ansprüche 1 bis 5, **dadurch gekennzeichnet, dass** die vorbestimmte Geschwindigkeit 10 km/h beträgt und das vorbestimmte Verhältnis 1/6 beträgt.

7. Verfahren nach einem der Ansprüche 1 bis 6, **dadurch gekennzeichnet, dass** die Variationsschwelle jedes der Trainingsmodi 10 % beträgt.

8. Detektionsvorrichtung zum Bestimmen der Müdigkeit eines Benutzers, die Folgendes umfasst:
eine Herzfrequenzmesseinheit (1), die so konfiguriert ist, dass sie die Herzfrequenz des Benutzers zu aufeinanderfolgenden Zeitpunkten misst, um eine Vielzahl von Herzfrequenzmessungen zu erzielen;
eine Positionierungseinheit (3), die so konfiguriert ist, dass sie erkennt, wo sich der Benutzer zu aufeinanderfolgenden Zeitpunkten befindet, um eine Vielzahl von Positionen zu erhalten, und ferner so konfiguriert, dass sie basierend auf den Positionen eine Vielzahl von Geschwindigkeitsmessungen des Benutzers berechnet;
eine Display-Einheit (4); und
eine Verarbeitungseinheit (5), die mit der besagten Herzfrequenzmesseinheit (1), der besagten Positionierungseinheit (3) und der besagten Display-Einheit (4) elektrisch verbunden ist und eine Vielzahl von Trainingsmodi und eine Vielzahl von ersten Bereichen von Herzfrequenz-bezogenen Verhältnissen speichert, wobei jeder der Trainingsmodi durch einen ersten Bereich von Herzfrequenz-bezogenen Verhältnissen definiert ist, und
eine Variationsschwelle und einen vorbestimmten Trainingszeitraum, wobei die besagte Verarbeitungseinheit (5) so konfiguriert ist, dass sie ein Verfahren zum Bestimmen der Müdigkeit implementiert, das die folgenden Schritte enthält:
a) Erhalten der Herzfrequenzmessungen des Benutzers von der besagten Herzfrequenzmesseinheit (1), und
b) Berechnen eines Verhältnisses eines resultierenden Werts der Herzfrequenzmessung abzüglich einer Ruheherzfrequenz des Benutzers zu einem resultierenden Wert einer maximalen Herzfrequenz des Benutzers abzüglich der Ruheherzfrequenz für jede der Herzfrequenzmessungen, um als Referenzverhältnis zu dienen,
**dadurch gekennzeichnet, dass** das von der besagten Verarbeitungseinheit (5) implementierte Verfahren zum Bestimmen der Müdigkeit nach Schritt b ferner die folgenden Schritte umfasst:
c) Auswählen eines der Trainingsmodi basierend auf dem zuletzt berechneten Referenzverhältnis,
d) Erhalten einer Vielzahl von Geschwindigkeitsmessungen, die nacheinander von der besagten Positionierungseinheit (3) über einen Zeitraum gemessen wurden, der dem vorbestimmten Trainingszeitraum des in Schritt c ausgewählten Trainingsmodus entspricht, von der besagten Positionierungseinheit (3),
e) Berechnen eines Durchschnittswerts der Geschwindigkeitsmessungen, um als Durchschnittsgeschwindigkeit zu dienen,
f) Dividieren einer Anzahl derjenigen der Geschwindigkeitsmessungen, die um mindestens ein Variationsverhältnis größer oder kleiner als die Durchschnittsgeschwindigkeit sind, durch eine Gesamtzahl der Geschwindigkeitsmessungen, um ein abnormales Geschwindigkeitsverhältnis zu erhalten,
g) Bestimmen, ob die Durchschnittsgeschwindigkeit größer als eine vorbestimmte Geschwindigkeit ist und ob das abnormale Geschwindigkeitsverhältnis kleiner als ein vorbestimmtes Verhältnis ist,
h) wenn die Bestimmung, ob die Durchschnittsgeschwindigkeit größer als die vorbestimmte Geschwindigkeit ist, und die Bestimmung, ob das abnormale Geschwindigkeitsverhältnis kleiner als das vorbestimmte Verhältnis ist, beide positiv sind, Bestimmen, ob das zuletzt berechnete Referenzverhältnis um die Variationsschwelle des in Schritt c ausgewählten Trainingsmodus größer als ein zuvor berechnetes Referenzverhältnis ist, wobei das zuvor berechnete Referenzverhältnis das Referenzverhältnis ist, das zu einem früheren Zeitpunkt vor dem Zeitpunkt des Berechnens des letzten Referenzverhältnisses berechnet wurde und dem vorbestimmten Trainingszeitraum des in Schritt c ausgewählten Trainingsmodus entspricht, und
i) Steuern der besagten Display-Einheit (4) zum Ausgeben einer ersten Benachrichtigung, die anzeigt, dass der Benutzer während des Trainings müde ist, wenn die in Schritt h) gemachte Bestimmung positiv ist; und
ferner **gekennzeichnet durch** eine Beschleunigungsmesseinheit (2), die mit der besagten Verarbeitungseinheit (5) elektrisch verbunden und so konfiguriert ist, dass sie nacheinander Beschleunigungsmessungen des Benutzers misst, wobei das von der besagten Verarbeitungseinheit (5) implementierte Verfahren zum Bestimmen der Müdigkeit vor Schritt c ferner die folgenden Schritte enthält:
Erhalten einer Vielzahl von Beschleunigungsmessungen des Benutzers, die in einem vordefinierten Zeitraum gemessen wurden, von der besagten Beschleunigungsmesseinheit (2);
Bestimmen, ob alle Beschleunigungsmessungen in dem vordefinierten Zeitraum kleiner als eine vorbestimmte Beschleunigungsschwelle sind;
wenn bestimmt wird, dass alle Beschleunigungsmessungen in dem vordefinierten Zeitraum kleiner als die vorbestimmte Beschleunigungsschwelle sind,
Bestimmen der Müdigkeit des Benutzers während der Ruhephase und Steuern der besagten Display-Einheit (4) zum Ausgeben einer entsprechenden zweiten Benachrichtigung; und
wenn bestimmt wird, dass nicht alle Beschleunigungsmessungen in dem vordefinierten Zeitraum kleiner als die vorbestimmte Beschleunigungsschwelle sind, Ausführen der Schritte c bis i des Verfahrens zum Bestimmen der Müdigkeit.

9. Detektionsvorrichtung nach Anspruch 8, **dadurch gekennzeichnet, dass** die besagte Verarbeitungseinheit (5) ferner eine Vielzahl von Müdigkeitsgraden und eine Vielzahl von zweiten Bereichen von Herzfrequenz-bezogenen Verhältnissen speichert, wobei jeder Müdigkeitsgrad durch einen zweiten Bereich von Herzfrequenz-bezogenen Verhältnissen definiert ist, und so konfiguriert ist, dass sie die Ermüdung des Benutzers durch Folgendes bestimmt:
Bestimmen, in welchen der zweiten Bereiche von Herzfrequenz-bezogenen Verhältnissen das zuletzt berechnete Referenzverhältnis fällt; und
Bestimmen, dass sich der Benutzer in einem der Müdigkeitsgrade befindet, der einem der zweiten Bereiche von Herzfrequenz-bezogenen Verhältnissen entspricht, in die das zuletzt berechnete Referenzverhältnis fällt, und Ausgeben der zweiten Benachrichtigung, die dem besagten einen der Müdigkeitsgrade entspricht.

10. Detektionsvorrichtung nach einem der Ansprüche 8 bis 9, **dadurch gekennzeichnet, dass** der vordefinierte Zeitraum 20 Minuten beträgt.

11. Detektionsvorrichtung nach einem der Ansprüche 8 bis 10, **dadurch gekennzeichnet, dass** die vorbestimmte Beschleunigungsschwelle 20 cm/s² beträgt.

12. Detektionsvorrichtung nach einem der Ansprüche 8 bis 11, **dadurch gekennzeichnet, dass** das Variationsverhältnis 20 % beträgt.

13. Detektionsvorrichtung nach einem der Ansprüche 8 bis 12, **dadurch gekennzeichnet, dass** die vorbestimmte Geschwindigkeit 10 km/h beträgt und das vorbestimmte Verhältnis 1/6 beträgt.

14. Detektionsvorrichtung nach einem der Ansprüche 8 bis 13, **dadurch gekennzeichnet, dass** die Variationsschwelle jedes der Trainingsmodi 10 % beträgt.

## Revendications

1. Procédé mis en œuvre par un processeur pour déterminer la fatigue d'un utilisateur, pour être mis en œuvre par un dispositif de détection qui stocke une pluralité de modes d'exercice et une pluralité de premières plages de ratios liés à la fréquence cardiaque, chacun des modes d'exercice étant défini par une première plage de ratios liés à la fréquence cardiaque et ayant un seuil de variation et une période de temps d'exercice prédéterminée, le procédé comprenant les étapes suivantes :
A) obtenir, à partir d'une unité de mesure de fréquence cardiaque (1), une fréquence cardiaque de l'utilisateur à des instants successifs afin d'obtenir une pluralité de mesures de fréquence cardiaque (S1) ; et
B) pour chacune des mesures de fréquence cardiaque, calculer un rapport entre une valeur résultante de la mesure de fréquence cardiaque moins une fréquence cardiaque au repos de l'utilisateur et une valeur résultante d'une fréquence cardiaque maximale de l'utilisateur moins la fréquence cardiaque au repos pour servir de rapport de référence (S02) ;
**caractérisé en ce que** le procédé comprend en outre les étapes suivantes après l'étape B :
C) sélectionner l'un des modes d'exercice sur la base du dernier rapport de référence calculé (S51) ;
D) obtenir, d'une unité de positionnement (3), une pluralité de mesures de vitesse de l'utilisateur qui ont été mesurées successivement sur une période de temps correspondant à la période de temps d'exercice prédéterminée du mode d'exercice sélectionné à l'étape C (S52) ;
E) calculer une moyenne des mesures de vitesse pour servir de vitesse moyenne (S53) ;
F) diviser un certain nombre de ces mesures de vitesse qui sont supérieures ou inférieures à la vitesse moyenne au moins par un rapport de variation par le nombre total des mesures de vitesse pour obtenir un rapport de vitesse anormal (S54) ;
G) déterminer si la vitesse moyenne est supérieure à une vitesse prédéterminée et si le rapport de vitesse anormal est inférieur à un rapport prédéterminé (S55) ;
H) lorsque la détermination visant à savoir si la vitesse moyenne est supérieure à la vitesse prédéterminée et la détermination visant à savoir si le rapport de vitesse anormal est inférieur au rapport prédéterminé sont toutes deux affirmatives, déterminer si le dernier rapport de référence calculé est supérieur à un rapport de référence calculé précédemment par le seuil de variation du mode d'exercice sélectionné à l'étape C, le rapport de référence calculé précédemment étant le rapport de référence calculé à un moment antérieur au moment du calcul du dernier rapport de référence et correspondant à la période de temps d'exercice prédéterminée du mode d'exercice sélectionné à l'étape C (S56) ; et
I) émettre, sur une unité d'affichage (4), une première notification indiquant que l'utilisateur est fatigué pendant l'exercice lorsque la détermination effectuée à l'étape H) est affirmative (S57) ;
le procédé comprenant en outre les étapes suivantes avant l'étape C :
J) obtenir, d'une unité de mesure d'accélération (2), une pluralité de mesures d'accélération de l'utilisateur dans une période de temps prédéfinie (S3) ;
K) déterminer si toutes les mesures d'accélération dans la période de temps prédéfinie sont inférieures à un seuil d'accélération prédéterminé (S4) ;
L) lorsqu'il est déterminé que toutes les mesures d'accélération dans la période de temps prédéfinie sont inférieures au seuil d'accélération prédéterminé, déterminer la fatigue de l'utilisateur pendant le repos et émettre une deuxième notification correspondante (S6) ; et
M) lorsqu'il est déterminé que les mesures d'accélération dans la période de temps prédéfinie ne sont pas toutes inférieures au seuil d'accélération prédéterminé, exécuter les étapes C à I.

2. Procédé selon la revendication 1, le dispositif de détection stockant en outre une pluralité de niveaux de fatigue et une pluralité de deuxièmes plages de ratios liés à la fréquence cardiaque, chaque niveau de fatigue étant défini par une deuxième plage de ratios liés à la fréquence cardiaque, le procédé étant **caractérisé en ce que** l'étape L) inclut :
déterminer dans laquelle des deuxièmes plages de ratios liés à la fréquence cardiaque se situe le dernier rapport de référence calculé (S61) ; et
déterminer que l'utilisateur se trouve à l'un des niveaux de fatigue qui correspond à l'une des deuxièmes plages de ratios liés à la fréquence cardiaque dans laquelle se situe le dernier rapport de référence calculé (S62).

3. Procédé selon l'une quelconque des revendications 1 à 2, **caractérisé en ce que** la période de temps prédéfinie est de 20 minutes.

4. Procédé selon l'une quelconque des revendications 1 à 3, **caractérisé en ce que** le seuil d'accélération prédéterminé est de 20 cm/s².

5. Procédé selon l'une quelconque des revendications 1 à 4, **caractérisé en ce que** le rapport de variation est de 20 %.

6. Procédé selon l'une quelconque des revendications 1 à 5, **caractérisé en ce que** la vitesse prédéterminée est de 10 km/h et le rapport prédéterminé est de 1/6.

7. Procédé selon l'une quelconque des revendications 1 à 6, **caractérisé en ce que** le seuil de variation de chacun des modes d'exercice est de 10 %.

8. Dispositif de détection pour déterminer la fatigue d'un utilisateur, comprenant :
une unité de mesure de fréquence cardiaque (1) configurée pour mesurer la fréquence cardiaque de l'utilisateur à des instants successifs afin d'obtenir une pluralité de mesures de fréquence cardiaque ;
une unité de positionnement (3) configurée pour détecter où se trouve l'utilisateur à des instants successifs afin d'obtenir une pluralité de positions, et configurée en outre pour calculer une pluralité de mesures de vitesse de l'utilisateur sur la base des positions ;
une unité d'affichage (4) ; et
une unité de traitement (5) connectée électriquement à ladite unité de mesure de fréquence cardiaque (1), à ladite unité de positionnement (3), et à ladite unité d'affichage (4), et stockant une pluralité de modes d'exercice et une pluralité de premières plages de ratios liés à la fréquence cardiaque, chacun des modes d'exercice étant défini par une première plage de ratios liés à la fréquence cardiaque, et
un seuil de variation et une période de temps d'exercice prédéterminée, ladite unité de traitement (5) étant configurée pour mettre en œuvre une procédure de détermination de la fatigue qui inclut les étapes suivantes
a) obtenir les mesures de fréquence cardiaque de l'utilisateur à partir de ladite unité de mesure de fréquence cardiaque (1), et
b) pour chacune des mesures de fréquence cardiaque, calculer un rapport entre la valeur résultante de la mesure de fréquence cardiaque moins la fréquence cardiaque au repos de l'utilisateur et la valeur résultante de la fréquence cardiaque maximale de l'utilisateur moins la fréquence cardiaque au repos pour servir de rapport de référence,
**caractérisé en ce que** la procédure de détermination de la fatigue mise en œuvre par ladite unité de traitement (5) comprend en outre les étapes suivantes après l'étape b :
c) sélectionner l'un des modes d'exercice sur la base du dernier rapport de référence calculé,
d) obtenir, à partir de ladite unité de positionnement (3), une pluralité de mesures de vitesse qui ont été mesurées successivement par ladite unité de positionnement (3) sur une période de temps correspondant à la période de temps d'exercice prédéterminée du mode d'exercice sélectionné à l'étape c,
e) calculer une moyenne des mesures de vitesse pour servir de vitesse moyenne,
f) diviser un certain nombre de ces mesures de vitesse qui sont supérieures ou inférieures à la vitesse moyenne au moins par un rapport de variation par le nombre total de mesures de vitesse pour obtenir un rapport de vitesse anormal,
g) déterminer si la vitesse moyenne est supérieure à une vitesse prédéterminée et si le rapport de vitesse anormal est inférieur à un rapport prédéterminé,
h) lorsque la détermination visant à établir si la vitesse moyenne est supérieure à la vitesse prédéterminée et la détermination visant à établir si le rapport de vitesse anormal est inférieur au rapport prédéterminé sont toutes deux affirmatives, déterminer si le dernier rapport de référence calculé est supérieur à un rapport de référence précédemment calculé par le seuil de variation du mode d'exercice sélectionné à l'étape c, le rapport de référence précédemment calculé étant le rapport de référence calculé à un moment antérieur au moment du calcul du dernier rapport de référence et correspondant à la période de temps d'exercice prédéterminée du mode d'exercice sélectionné à l'étape c, et
i) commander à ladite unité d'affichage (4) d'émettre une première notification indiquant que l'utilisateur est fatigué pendant l'exercice lorsque la détermination effectuée à l'étape h) est affirmative ; et
**caractérisé en outre par** une unité de mesure d'accélération (2) connectée électriquement à ladite unité de traitement (5) et configurée pour mesurer successivement les mesures d'accélération de l'utilisateur, la procédure de détermination de la fatigue mise en œuvre par ladite unité de traitement (5) inclut en outre, avant l'étape c, les étapes suivantes :
obtenir, à partir de ladite unité de mesure d'accélération (2), une pluralité de mesures d'accélération de l'utilisateur mesurées pendant une période de temps prédéfinie ;
déterminer si toutes les mesures d'accélération pendant la période de temps prédéfinie sont inférieures à un seuil d'accélération prédéterminé ;
lorsqu'il est déterminé que toutes les mesures d'accélération pendant la période de temps prédéfinie sont inférieures au seuil d'accélération prédéterminé, déterminer la fatigue de l'utilisateur pendant le repos et commander à ladite unité d'affichage (4) d'émettre une deuxième notification correspondante ; et
lorsqu'il est déterminé que les mesures d'accélération dans la période de temps prédéfinie ne sont pas toutes inférieures au seuil d'accélération prédéterminé, exécuter les étapes c à i de la procédure de détermination de la fatigue.

9. Dispositif de détection selon la revendication 8, **caractérisé en ce que** ladite unité de traitement (5) stocke en outre une pluralité de niveaux de fatigue et une pluralité de deuxièmes plages de ratios liés à la fréquence cardiaque, chaque niveau de fatigue étant défini par une deuxième plage de ratios liés à la fréquence cardiaque, et est configurée pour déterminer la fatigue de l'utilisateur en :
déterminant dans laquelle des deuxièmes plages de ratios liés à la fréquence cardiaque se situe le dernier rapport de référence calculé ; et
déterminant que l'utilisateur se trouve à l'un des niveaux de fatigue qui correspond à l'une des deuxièmes plages de ratios liés à la fréquence cardiaque, dans laquelle se situe le dernier rapport de référence calculé, et émettant la deuxième notification qui correspond audit l'un des niveaux de fatigue.

10. Dispositif de détection selon l'une quelconque des revendications 8 à 9, **caractérisé en ce que** la période de temps prédéfinie est de 20 minutes.

11. Dispositif de détection selon l'une quelconque des revendications 8 à 10, **caractérisé en ce que** le seuil d'accélération prédéterminé est de 20 cm/s².

12. Dispositif de détection selon l'une quelconque des revendications 8 à 11, **caractérisé en ce que** le rapport de variation est de 20 %.

13. Dispositif de détection selon l'une quelconque des revendications 8 à 12, **caractérisé en ce que** la vitesse prédéterminée est de 10 km/h et le rapport prédéterminé est de 1/6.

14. Dispositif de détection selon l'une quelconque des revendications 8 à 13, **caractérisé en ce que** le seuil de variation de chacun des modes d'exercice est de 10 %.
